# EUROPÄISCHE PATENTSCHRIFT

(11) **EP 0 452 631 B1**
(45) Veröffentlichungstag und Bekanntmachung des Hinweises auf die Patenterteilung: **07.12.1994**
(21) Anmeldenummer: 91102590.6
(22) Anmeldetag: 22.02.1991
(51) Int. Cl.: A61B 17/22

(54) **Dilatationskatheter**
Dilatation catheter
Cathéter de dilatation

(30) Priorität: 20.04.1990 DE 4012649
(43) Veröffentlichungstag der Anmeldung: 23.10.1991
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Patent- und Rechtsanwaltssozietät, Schmitt, Maucher & Börjes

(56) Entgegenhaltungen:
- EP-A- 0 177 782
- EP-A- 0 267 539
- WO-A-89/01800
- WO-A-90/03760
- US-A- 4 854 330

## Beschreibung

Die Erfindung betrifft einen Dilatationskatheter zum Vermindern oder Beseitigen von Gefäßverengungen oder Gefäßverschlüssen mit einem an seinem distalen Ende angebrachten Druckkörper, der zum Ausüben eines Druckes auf die eingeengte Gefäßwand dient.

Die Dilatation verengter Gefäße wird derzeit hauptsächlich mit sogenannten Ballon-Kathetern durchgeführt (vgl. z.B. WO-A-8 901 800). Dabei besteht die Gefahr, daß die Gefäße durch die Aufweitung des Ballons überdehnt werden können, so daß Risse in den Gefäßwänden entstehen können, die dann eine Ursache für eine erneute Stenose bilden können.

Es sind deshalb auch schon rotierende Katheter zur Dilatation von Gefäßen bekanntgeworden, die einen mit Diamantstaub beschichteten Metallkopf als Druckkörper haben, der über eine flexible Achse mit hoher Drehzahl angetrieben werden kann, um innerhalb der Stenose das Innenlumen durch Abschleifen der Ablagerungen zu vergrößern. Dabei besteht eine erhebliche Gefahr darin, daß durch dieses "blinde Schleifen" auch gesunde Teile der Gefäßwand verletzt oder gar perforiert werden können.

Aus der US-PS 4 030 503 ist ein drehbarer Katheter für eine Dilatation bekannt, der schwierig zu handhaben ist und bei welchem die Gefahr besteht, daß Partikel aus der Gefäßverengung abgelöst werden und anschließend in die Blutbahn gelangen und damit eine Emboliegefahr bilden.

Aus der DE-PS 37 32 236 ist ein Rekanalisationskatheter bekannt, bei welchem die Gefäßverengung durch Fräsen entfernt werden soll, was ebenfalls die vorerwähnte Gefahr der Ablösung von Partikeln und der daraus resultierenden Emboliegefahr mit sich bringt und auch eine Verletzungsgefahr für die Gefäßwände darstellt.

Es besteht deshalb die Aufgabe, einen Dilatationskatheter der eingangs erwähnten Art zu schaffen, mit welchem mit Hilfe eines Druckkörpers und einer Rotations- oder kreisenden Bewegung das Gewebe in der Stenose radial nach außen verdichtet werden kann, ohne daß sich Partikel ablösen können. Es soll durch eine schonende Verdichtung und Glättung der Gefäßinnenwände eine möglichst geringe Restenosierungsrate erreicht werden können.

Die Lösung dieser Aufgabe besteht im wesentlichen darin, daß der Katheter einen inneren Führungskanal für ein Stilett aufweist und nahe dem Druckkörper flexibel biegbar oder auslenkbar ist, daß das Stilett als Stütz- und Steuerelement nahe seinem in Gebrauchsstellung bis zu oder in den Druckkörper ragenden Ende eine Vorbiegung oder Vorkrümmung aufweist und daß das Stilett mit einem außerhalb der Gefäßes am proximalen Ende angeordneten Drehantrieb kuppelbar und dadurch drehantreibbar ist.

Es kann also das Stilett in das Innere des biegsamen Katheters eingeführt werden, wobei seine distale Spitze so verformt ist, daß sie auch den Katheter mit dem Druckkörper, bevorzugt einem etwa olivenartigen Körper, in eine kreisende Bewegung versetzen kann, die einer Rotationsbewegung überlagert sein kann. Wird das an seiner Spitze vorgebogene oder vorgekrümmte Stilett zusammen mit dem rotierenden biegsamen Katheter in ein Gefäß eingeführt, nimmt dieses die Form des gebogenen Stilettes an. Leichte steuernde manuelle Drehbewegungen gestatten es dabei, die Spitze des Katheters bei Abzweigungen in das gewünschte Gefäß oder die gewünschte Arterie zu dirigieren. In dieser Position könnte man beispielsweise den verschlossenen Ast einer Y-förmig aufgeteilten Arterie durch rotierende und schiebenede Bewegungen des Katheters wieder öffnen. Handelt es sich um eine stenosierte Arterie, kann die distale Spitze des biegsamen Katheters bis an die Stenose herangeführt werden. Danach können sowohl der Katheter als auch das Stilett zur Rotation gebracht werden, so daß der Druckkörper nicht nur eine Rotationsbewegung, sondern gleichzeitig noch eine kreisförmige Bewegung mit Druck auf das stenosierte Gewebe durchführt und es radial verdichtet. Je nach Größe und Stärke der Vorkrümmung des distalen Endes des Stilettes kann der Radius dieser exzentrischen oder Kreisbewegung des Druckkörpers verändert werden.

Besonders zweckmäßig ist es dabei, wenn die bleibende Vorkrümmung oder Vorbiegung des Stilettes - gegebenenfalls wiederholt durch stärkere Verbiegung oder Zurückbiegung - veränderbar ist. Es kann also an ein und demselben Stilett die vorerwähnte Veränderung des Radius der Exzentrizität des Druckkörpers auf den jeweiligen Benutzungsfall abgestimmt werden.

Der Drehantrieb kann ein Motor mit einer hohlen Abtriebswelle sein, der Katheter kann durch diese hohle Abtriebswelle und den Motor hindurch verlaufen, mit der Abtriebswelle drehfest kuppelbar sein und das Stilett kann nach dem Einführen in den hohlen Katheter mit dessen proximalen Ende drehfest kuppelbar sein. Dies stellt eine besonders einfache Möglichkeit dar, zunächst einmal nur mit rotierendem Katheter dessen Druckkörper an die Stenose heranzuführen, wobei das Stilett bereits entsprechende Führungsaufgaben übernehmen kann, wonach dieses ebenfalls drehfest - in einfache Weise mit dem rotierbaren Katheter - gekuppelt werden kann, um dann eine synchrone Drehbewegung mit dem Katheter durchzuführen, wobei die Vorkrümmung oder Biegung des Stilettes in der schon erwähnten Weise zum Tragen kommt, daß der Druckkörper nicht nur um seine eigene, sondern auch noch um eine exzentrische Achse bewegt wird.

Eine abgewandelte Ausführungsform, die auch ein alleiniges Rotieren des Stilettes mit entsprechender kreisender Bewegung des Druckkörpers ohne dessen Eigenrotation erlaubt, kann darin bestehen, daß ein Antriebsmotor für den Drehantrieb des Katheters und des Stilettes zwei in Flucht miteinander angeordnete Abtriebswellen hat, die hohl sind, wobei das Stilett durch beide Abtriebswellen in den mit der ersten Abtriebswelle drehfest gekuppelten Katheter einführbar und in Gebrauchsstellung mit der zweiten Abtriebswelle lösbar kuppelbar ist. Wird nur das Stilett mit dem Antrieb gekuppelt, macht der Druckkörper lediglich eine kreisende exzentrische Bewegung, während bei alleiniger Kupplung des Katheters mit dem Drehantrieb der Druckkörper um seine eigene, eventuell durch das eingeführte Stilett seitlich ausgebogene Achse durchführt. Werden beide Teile mit dem Drehantrieb gekuppelt, findet die synchrone Drehbewegung des Katheters und des Stilettes bei gleichzeitiger kreisender Bewegung des Druckkörpers statt. Selbstverständlich könnte diese separate Drehbarkeit von Stilett und Katheter auch mit zwei einzelnen Antrieben erreicht werden, die dann aber eventuell Schwierigkeiten machen, wenn Stilett und Katheter synchron angetrieben werden sollen.

Das distale Ende des Stilettes kann einen Anschlag, beispielsweise eine Kugel oder dergleichen zum Festlegen gegen einen am distalen Ende, insbesondere im Inneren des Druckkörpers befindlichen Gegenanschlag oder Absatz aufweisen. Somit kann sichergestellt werden, daß das Stilett bei Erreichen dieses Anschlages auch mit seiner Vorkrümmung an der richtigen vorgewählten flexiblen Stelle des Katheters zu liegen kommt. Gleichzeitig wird verhindert, daß das Stilett ungewollt aus dem Katheter am distalen Ende austritt.

Unter Umständen kann es erwünscht sein, daß das Stilett beim Einführen des Katheters oder in den Katheter ungebogen und gerade ist. In Gebrauchsstellung soll es aber die vorerwähnte Vorbiegung oder Vorkrümmung haben. Diese scheinbar widersinnige Forderung kann dadurch erfüllt werden, daß das Stilett bei Normaltemperatur gerade ist, zumindest aber im Bereich seines distalen Endes am Ort seiner Vorbiegung oder Vorkrümmung aus Memory-Metall besteht und bei Körpertemperatur gebogen oder gekrümmt ist. Somit kann das Stilett in gerader Lage eingeführt werden, befindet sich dann innerhalb des Körpers und ist dort der entsprechenden Körpertemperatur ausgesetzt, so daß das Memory-Metall die entsprechende Krümmung einnimmt.

Eine weitere besonders zweckmäßige und vorteilhafte Ausgestaltung der Erfindung kann darin bestehen, daß das Stilett oder dergleichen in einer gegenüber dem distalen Ende etwas zurückgezogenen Position mit dem Drehantrieb kuppelbar ist und sein durch die Vorkrümmung ausgelenktes Ende den den Druckkörper des Katheters benachbarten biegsamen oder flexiblen Bereich des Katheters gegenüber dessen Verlauf und gegenüber der mittleren Position des Druckkörpers aus lenkt und bei Rotation des Stilettes in eine etwa kreisförmige Bewegung versetzt, und daß an diesem Bereich vorzugsweise ein zweiter Druckkörper an der Außenseite des Katheters fest oder lösbar angeordnet ist. Mit einer solchen Anordnung kann erreicht werden, daß der distale Druckkörper sich zunächst einen Weg durch eine Stenose bahnt, wonach ihm ein Druckkörper folgt, der auf einer größeren Kreisbahn umläuft und dabei gleichzeitig synchron mit dem Stilett rotiert. Es kann aber auch nur das Stilett alleine rotieren und mit Abstand zu dem ersten Druckkörper eine Aufweitung eines Gefäßes durch diesen kreisenden zweiten Druckkörper bewirken.

Der Katheter kann eine einlagige und vorzugsweise ein- oder mehrgängige Wendel aus flexiblem Metalldraht, von Kunststofffäden oder -bändern sein. Er bildet auf diese Weise automatisch den inneren Kanal für das Stilett aus. Gleichzeitig stellt er auf diese Weise eine effektive biegsame Welle dar und kann auf die erforderliche Flexibilität insbesondere nahe seinem distalen Ende und benachbart zu dem Druckkörper eingestellt sein.

Die nebeneinanderliegenden schraubenlinienförmigen Windungen können dabei zumindest in den geraden Bereichen und an der Innenseite einer Krümmung einander berühren. Es ergibt sich so eine ausreichede Drehfestigkeit und ein im wesentlichen geschlossener innerer Kanal für das Stilett.

Falls die Drehfestigkeit erhöht werden soll oder auch gegensinnige Rotationen einschaltbar und einstellbar sein sollen, also der Druckkörper mal im mal gegen den Uhrzeigersinn angetrieben werden können soll, kann es zweckmäßig sein, wenn der Katheter aus mehreren parallel gewundenen und sich kreuzenden Drähten, Fäden und/oder Bändern geflochten ist.

Insgesamt ergibt sich ein Katheter, mit dem durch eine rotierende Bewegung eines Druckkörpers oder auch zweier Druckkörper die Innenwand eines Gefäßes schonend erweitert und verdichtet und geglättet werden kann, ohne daß der Blutstrom im Gefäß blockiert wird, denn dieser kann seitlich und auch im Inneren des Katheters durch den für das Stilett bestimmten Kanal weiterströmen. Dabei hat die Vorkrümmung des Stilettes insofern eine Mehrfachfunktion, als bei nur eingeschobenem, aber nicht drehangetriebenem Stilett der Druckkörper gut in Abzweigungen dirigiert werden kann, wobei er auch selbst um seine nun seitlich ausgelenkte Achse rotieren kann, was unter Umständen auch schon zur Aufweitung eines Gefäßes unmittelbar an oder hinter einer Abzweigung zweckmäßig ist; wird die biegsame Welle und das Stilett synchron angetrieben, macht der Druckkörper zu seiner Eigendrehbewegung gleichzeitig noch eine exzentrische oder kreisende Bewegung, um die ursprüngliche Mittelachse und schließlich besteht sogar die Möglichkeit, nur das Stilett drehanzutreiben und den distalen Druckkörper oder einen demgegenüber zurückversetzten Bereich oder Druckkörper in eine entsprechende kreisende Bewegung zu versetzen. Somit sind dem Benutzer eine Mehrzahl von Möglichkeiten geboten, der jeweiligen Stenose in einem Gefäß so effektiv und gleichzeitig so schonend wie möglich beizukommen.

Dabei besteht sogar die Möglichkeit, in Einzelfällen, insbesondere bei engen Gefäßen, das Stilett aus seiner vorgekrümmten Position wieder geradezubiegen oder von vorneherein ein gerades Stilett einzusetzen, bis allein aufgrund der Rotationsbewegung des Druckkörpers eine genügende Erweiterung geschaffen ist, wonach das Stilett wieder herausgezogen, vorgebogen oder durch ein vorgebogenes ersetzt und wieder eingeführt werden kann, um nun auf einem größeren Umfang eine zusätzliche Erweiterung vorzunehmen.

Nachstehend ist die Erfindung mit ihren ihr als wesentlich zugehörenden Einzelheiten anhand der Zeichnung noch näher beschrieben. Es zeigt in zum Teil schematisierter Darstellung:
- Fig. 1: einen Drehantrieb für einen drehbaren Katheter mit einem in dessem Inneren eingeschobenen, vorgebogenen Stilett, wobei der Katheter in Kupplungsposition, das Stilett aber noch ausgekuppelt sind,
- Fig. 2: eine der Fig.1 entsprechende Darstellung, wobei der Katheter und das Stilett mit dem Antrieb drehfest gekuppelt sind,
- Fig. 3: das distale Ende eines Katheters mit eingeschobenem Stilett aus Memory-Metall noch in gerader Position, aber in Rotation,
- Fig. 4: eine der Fig.3 entsprechende Darstellung, wobei ein nahe dem distalen Ende unter einem stumpfen Winkel abgebogenes Stilett in den Führungskanal des Katheters eingeschoben ist, so daß dieses ausgelenkt ist und durch vorsichtige Drehbewegungen an dem Stilett auch nach der entgegengesetzten Richtung - insgesamt auf einer Kreisbahn - auslenkbar ist,
- Fig. 5: eine den Figuren 3 u.4 entsprechende Darstellung, wobei das vorgebogene, den Katheter auslenkende Stilett und der Katheter selbst rotieren, so daß das distale Ende des Katheters mit seinem Druckkörper eine kreisende Bewegung und eine Rotationsbewegung durchführen,
- Fig. 6: in etwas verkleinerter Darstellung das distale Ende des Katheters bei Annähern an eine Stenose, woraus die Größenverhältnisse deutlich werden, wonach vor allem der Katheter gegenüber dem Gefäß einen wesentlich geringeren Durchmesser haben kann und trotzdem aufgrund der Vorkrümmung mit Hilfe des Stilettes einen großen Durchmesserbereich - größer als ihn der Katheter selbst aufweist - bearbeiten kann,

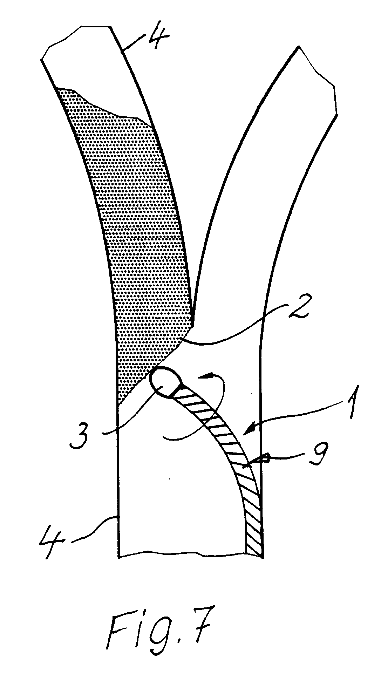

- Fig. 7: eine der Fig.6 entsprechende Darstellung, wobei eine Stenose in einer Gefäßabzweigung bearbeitet werden soll,
- Fig. 8: eine abgewandelte Ausführungsform des distalen Ende des Katheters, wobei dieser neben dem an seinem Ende befindlichen Druckkörper etwas zurückgesetzt einen weiteren Druckkörper aufweist, wobei das vorgebogene Stilett bis in den distalen Druckkörper vorgeschoben ist und seine Vorkrümmung zwischen beiden Druckkörpern liegt, sowie
- Fig. 9: eine der Fig. 8 entsprechende Darstellung, wobei das Stilett mit seinem distalen Ende innerhalb des zurückversetzten Druckkörpers angeordnet ist und aufgrund der Vorkrümmung des Stilettes diesen Druckkörper bei Rotation des Stilettes auf einer Kreisbahn exzentrisch zu der eigentlichen Katheterachse entlang eine Hüllkreis bewegt, dessen Durchmesser größer als der des Katheters oder seines Druckkörpers ist, so daß auch über eine größere Länge reichende Stenosen gleichzeitig und nachhaltig bearbeitet werden können.

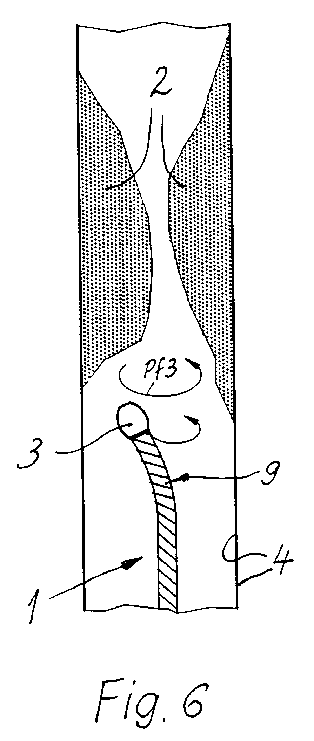

Ein Dilatationskatheter 1 dient zum Vermindern oder Beseitigen von Gefäßverengungen oder Gefäßverschlüssen 2 (vgl.Fig. 6 u.7) und hat dazu an seinem distalen Ende einen Druckkörper 3, der in allen Ausführungsbeispielen eine etwa olivenartige Form hat und mit seinem Außendurchmesser den Durchmesser des Katheters 1 übertrifft. Dieser Druckkörper 3 dient zum Ausüben eines Druckes auf die eingeengte Gefäßwand und ist mittels eines drehbaren Elementes, im wesentlichen des Katheters 1 drehantreibbar, wobei der Katheter 1 außerhalb des Gefäßes 4 am proximalen Ende mit einem dort angeordneten Drehantrieb 5 kuppelbar ist.

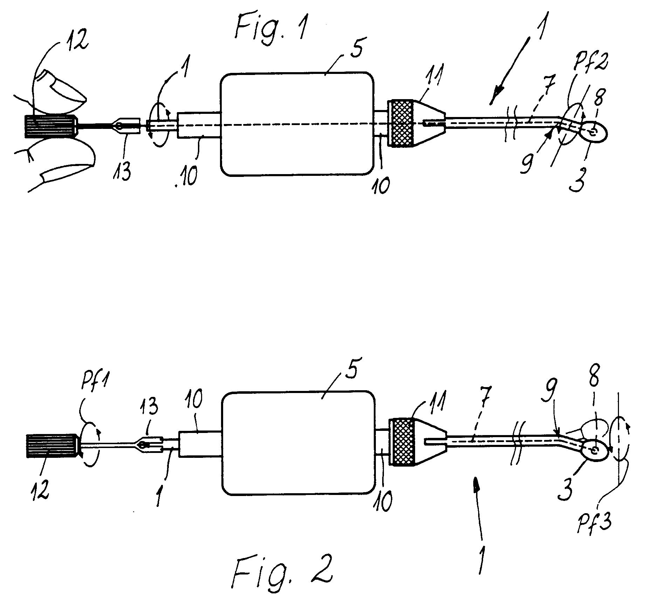

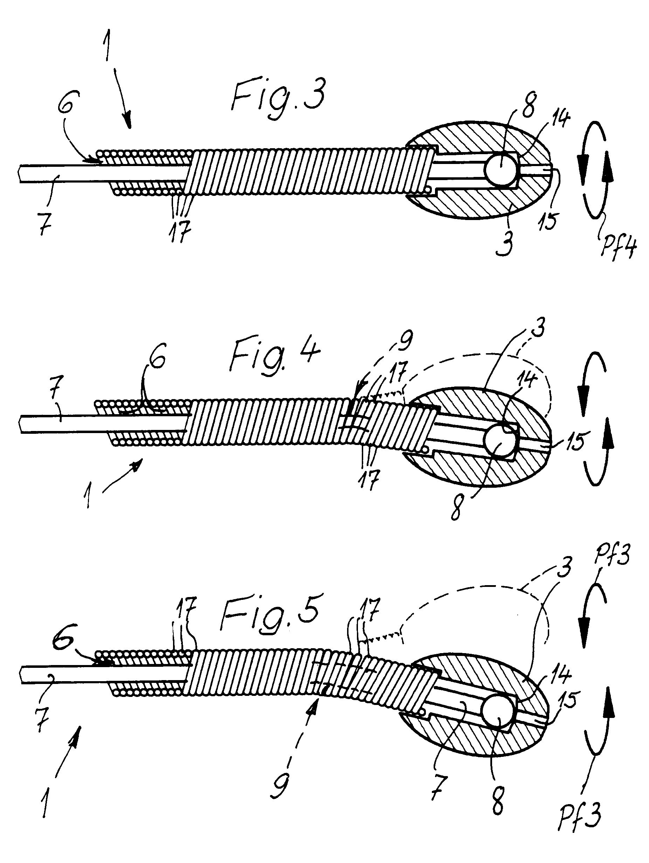

Wie vor allem in den Figuren 3 bis 5 sowie 8 und 9 ersichtlich, weist der Katheter 1 einen inneren Führungskanal 6 für ein Stilett 7 auf und ist nahe dem Druckkörper 3 flexibel biegbar und auslenkbar. Das Stilett 7 hat als Stütz- und Steuerelement nahe seinem in Gebrauchsstellung bis zu oder in den Druckkörper 3 ragenden distalen Ende 8 eine Vorbiegung oder Vorkrümmung 9, die durch das Einschieben in den Katheter 1 auf diesen übertragbar ist, so daß die Vorkrümmung 9 des Stilettes 7 in den Figuren 4 u.5 oder auch 8 u.9 sowie ferner in den Figuren 1 u.2 indirekt an einer entsprechenden Auslenkung des Katheters 1 nahe seinem distalen Ende benachbart zu dem Druckkörper 3 sichtbar wird. Auch das Stilett 7 ist mit einem Drehantrieb bzw. dem Drehantrieb 5 kuppelbar und kann somit in Drehrichtung angetrieben werden, wie es durch den Ringpfeil Pf 1 im linken Teil der Fig.2 angedeutet ist.

Die bleibende Vorkrümmung oder Vorbiegung 9 des Stilettes 7 kann veränderbar sein, indem dieses Stilett 7 im Bereich dieser Vorkrümmung 9 gegebenenfalls wiederholt stärker verbogen oder auch zurückgebogen wird. Es können aber auch zu einem Dilatationskatheter 1 unterschiedlich stark unter einem stumpfen Winkel vorgebogene oder vorgekrümmte Stilette 7 gehören, die wahlweise zum Einsatz kommen können.

Der Drehantrieb 5 ist ein Motor mit einer hohlen Abtriebswelle 10, durch welche der Katheter 1 hindurchverläuft und mit welcher er drehfest kuppelbar ist, wozu man in Fig. 1 u.2 eine entsprechende Spannvorrichtung 11 erkennt. In Fig. 1 u.2 ist angedeutet, daß dabei der Katheter 1 auch noch auf der entgegengesetzten Seite aus der hohlen Abtriebswelle 10 austritt, so daß dort das Stilett 7, dessen Handgriff 12 man in Fig. 1 u.2 erkennt, mit einer Klemme 13 drehfest angekuppelt werden kann, so daß dann Stilett 7 und Katheter 1 synchron von dem Antrieb 5 in Drehung versetzt werden können.

Es könnte allerdings auch eine Lösung vorgesehen sein, bei welcher ein Antriebsmotor 5 für den Drehantrieb des Katheters 1 und des Stilettes 7 - in ähnlicher Anordnung wie bei Fig. 1 u.2 - zwei in Flucht miteinander angeordnete Abtriebswellen 10 hat, die hohl sind, wobei das Stilett 7 durch beide Abtriebswellen 10 in den mit der ersten Abtriebswelle drehfest gekuppelten Katheter einführbar und in Gebrauchsstellung unmittelbar mit der zweiten Abtriebswelle lösbar kuppelbar ist.

Die Anordnung nach Fig.1 kann beispielsweise so lange beibehalten bleiben, bis die eigentliche Stenose oder Gefäßverengung 2 erreicht ist. Es kann also mit dem durch den vorgekrümmten Stilett 7 abgebogenen Katheter 1 der Weg durch die entsprechenden Gefäße und Abzweigungen gesucht werden, wobei der Katheter 1 auch schon gemäß dem Ringpfeil Pf 2 in dem rechten Teil der Fig.1 drehangetrieben sein kann. Ist nun ein Gefäßverschluß etwa gemäß Fig. 6 erreicht, dessen Querschnitt erheblich größer als der des Druckkörpers 3 ist, kann auch das Stilett 7 drehangetrieben werden, wonach der Druckkörper 3 nicht nur um seine eigene Achse rotiert, sondern gleichzeitig etwa auf einem Kreis gemäß dem Ringpfeil Pf 3 im rechten Teil der Fig.2 oder auch in Fig.5 umläuft, so daß trotz des relativ kleinen Eigenquerschnittes aufgrund dieser Exzentrizität des Druckkörpers 3, die er durch die stumpfwinklige Krümmung des Stilettes 7 erhält, ein wesentlich größerer Querschnitt eines Gefäßes 4 erreicht und bearbeitet werden kann.

Das distale Ende 8 des Stilettes 7 ist im Ausführungsbeispiel durch einen Anschlag in Form einer Kugel gebildet, der zum Festlegen gegen einen am distalen Ende, im Ausführungsbeispiel im Inneren des Druckkörpers 3 befindlichen Gegenanschlag oder Absatz 14 dient. Hinter diesem Absatz ist ein Durchtritt 15 durch den Druckkörper 3 erkennbare, so daß das Blut während der Betätigung des Katheters 1 dennoch auch durch diesen hindurch, vor allem aber auch an ihm vorbei, strömen kann.

Es sei noch erwähnt, daß das Stilett 7 bei Normaltemperatur gemäß Fig.3 gerade sein kann, im Bereich seines distalen Endes am Ort der Vorbiegung oder Vorkrümmung 9 jedoch aus Memory-Metall bestehen kann, so daß die Einheit aus Katheter 1 und Stilett 7 gegebenenfalls unter Rotation gemäß dem Ringpfeil Pf 4 in Fig. 3 geradlinig eingeschoben werden kann, sich dann aber aufgrund der entsprechenden Wahl des Memory-Metalles unter dem Einfluß der Körpertemperatur biegt und krümmt, wie es die Figuren 4 u.5 zeigen. Für den Fall, daß mit einer langwierigen Einführung gerechnet werden muß, kann eventuell dabei das Stilett 7 vorgekühlt sein, so daß es die Körpertemperatur erst so verzögert bekommt, daß es dann schon weitgehend eingeführt ist, wobei von Vorteil ist, daß das Stilett 7 sich dabei im Inneren des Katheters 1 befindet.

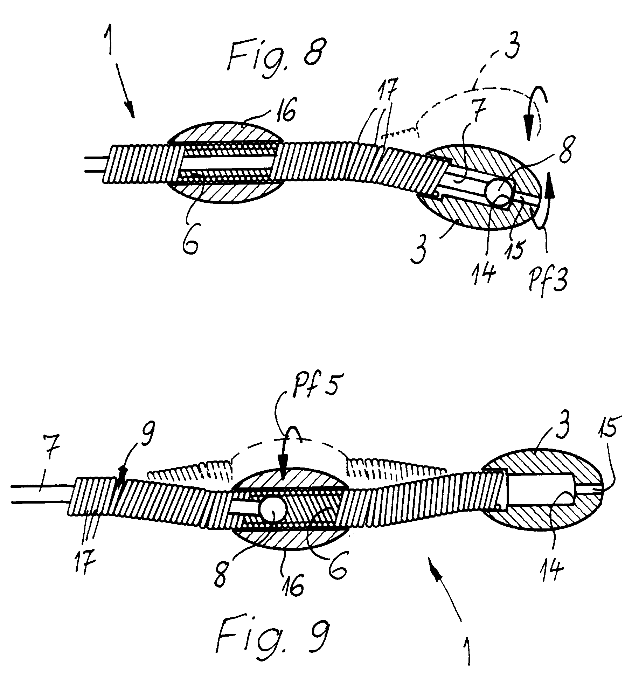

In den Figuren 8 u.9 ist dargestellt, daß das Stilett 7 in einer gegenüber dem distalen Ende etwas zurückgezogenen Position mit dem Drehantrieb 5 kuppelbar sein kann und sein durch seine Vorkrümmung ausgelenkter Bereich den dem Druckkörper 3 des Katheters 1 benachbarten biegsamen oder flexiblen Bereich des Katheters gegenüber dessen Verlauf und gegenüber der mittleren Position des Druckkörpers bzw. auch gegenüber diesem Druckkörper 3 auslenkt. In Fig. 8 befindet sich dabei das Stilett in vollständig eingeschobener Position, während Fig.9 die vorerwähnte Anordnung zeigt, bei der das distale Ende 8 des Stilettes 7 nicht den distalen Druckkörper 3, sondern einen diesem benachbarten Bereich des Katheters 1 auslenkt und bei Rotation des Stilettes 7 in eine kreisförmige Bewegung gemäß dem Pfeil Pf 5 versetzt. Dabei ist beim Ausführungsbeispiel nach Fig. 8 u.9 vorgesehen, daß an diesem Bereich ein zweiter Druckkörper 16 an der Außenseite des Katheters 1 fest oder gegebenenfalls lösbar abgeordnet sein kann, um den gewünschten Bearbeitungseffekt an einer Gefäßverengung 2 zu erreichen. Es ergibt sich durch diese Anordnung die Möglichkeit, eine über eine große Länge reichende Stenose zu bearbeiten, wobei wahlweise der distale Druckkörper 3 oder der demgegenüber zum proximalen Ende zurückversetzte Druckkörper 16 durch das vorgekrümmte Stilett 7 in eine Bewegung versetzt werden können, die über den eigenen Durchmesser hinausgeht. Dabei sind mehrere Kombinationen möglich, nämlich die Auslenkung mit Hilfe des Stilettes, jedoch lediglich eine Rotation des Katheters 1 oder aber die Rotation nur des Stilettes bei nicht rotierendem Katheter 1 und schließlich die bevorzugte und besonders effektive synchrone Drehbewegung von Stilett 7 und Katheter 1 mit seinem Druckkörper 16 und natürlich auch dem Druckkörper 3. Der Operateur hat also eine Vielzahl von Wahlmöglichkeiten, wie er nach und nach besonders schonend und dennoch effektiv einer Gefäßverengung 2 zu Leibe rücken kann.

In den Ausführungsbeispielen ist der Katheter 1 eine einlagige Wendel aus flexiblem Metalldraht. Auf diese Weise ergibt sich von selbst der Führungskanal 6 und noch genügend Platz zwischen dem Stilett 7 und der Innenwand dieses Kanales 6 für einen möglichst unbehinderten Blutfluß. Statt Drähten könnten auch Fäden oder Bänder vorhanden sein und je nach Anforderungen könnten diese auch mehrlagig und/oder mehrgängig angeordnet sein. Dabei berühren sich die nebeneinanderliegenden schraubenlinienförmigen Windungen 17 in den geraden Bereichen und an der Innenseite der jeweiligen Krümmungen. Somit ergibt sich eine gute Drehfestigkeit und auch eine gute Führung des Stilettes und seines kugeligen distalen Endes 8 beim Einführen.

Soll die Drehfestigkeit verstärkt werden, kann der Katheter auch aus mehreren parallel gewundenen und/oder sich kreuzenden Drähten, Fäden und/oder Bändern geflochten sein.

Der Dilatationskatheter 1 zum Vermindern oder Beseitigen von Gefäßverengungen oder Gefäßverschlüssen 2 hat an seinem distalen Ende wenigstens einen Druckkörper 3, eventuell aber beabstandet dazu auch noch einen zweiten Druckkörper 16. Der oder die Druckkörper dienen zum Ausüben eines Druckes auf die eingeengte Gefäßwand, um dort vorhandene Ablagerungen zurückzudrängen und so das Innenlumen des Gefäßes 4 wieder zu vergrößern. Dazu ist der Katheter 1 drehgelagert und am proximalen Ende mit einem Drehantrieb 5 kuppelbar.

Um einen größeren Umfang bzw. Querschnitt innerhalb eines Gefäßes 4 bearbeiten zu können, als er durch den oder die Druckkörper 3 bzw.16 vorgegeben ist, hat der Katheter 1 einen inneren Führungskanal 6 für ein Stilett 7 und ist nahe den Druckkörpern 3 u.16 flexibel biegsam oder auslenkbar. Das Stilett weist als Stütz- und Steuerelement nahe seinem in Gebrauchsstellung bis zu oder in den Druckkörper 3 oder den Druckkörper 16 ragenden Ende 8 eine Vorbiegung oder Vorkrümmung 9 auf und kann seinerseits mit einem Drehantrieb 5 gekuppelt und dadurch vorzugsweise synchron mit dem Katheter 1 drehangetrieben werden, so daß der oder die Druckkörper nicht nur um ihre eigene Achse, sondern gleichzeitig exzentrisch auf einer Kreisbahn bewegt werden.

## Patentansprüche

1. Dilatationskatheter (1) zum Vermindern oder Beseitigen von Gefäßverengungen oder Gefäßverschlüssen (2) mit einem an seinem diestalen Ende angebrachten Druckkörper (3), der zum Ausüben eines Druckes auf die eingeengte Gefäßwand dient, **dadurch gekennzeichnet**, daß der Katheter (1) einen inneren Führungskanal (6) für ein Stilett (7) aufweist und nahe dem Druckkörper (3) flexibel biegbar oder auslenkbar ist, daß das Stilett (7) als Stütz- und Steuerelement nahe seinem in Gebrauchsstellung bis zu oder in den Druckkörper (3) ragenden Ende (8) eine Vorbiegung oder Vorkrümmung (9) aufweist und daß das Stilett (7) mit einem außerhalb des Gefäßes am proximalen Ende angeordneten Drehantrieb (5) kuppelbar und dadurch drehantreibbar ist.

2. Katheter nach Anspruch 1, dadurch gekennzeichnet, daß die bleibende Vorkrümmung oder Vorbiegung (9) des Stilettes (7)- gegebenenfalls wiederholt durch stärkere Verbiegung oder Zurückbiegung - veränderbar ist.

3. Katheter nach Anspruch 1 oder 2, dadurch gekennzeichnet, daß der Drehantrieb (5) ein Motor mit einer hohlen Abtriebswelle (10) ist, daß der Katheter (1) durch diese hohler Abtriebswelle und den Motor hindurch verläuft, mit der Abtriebswelle drehfest kuppelbar ist und daß das Stilett nach dem Einführen in den hohlen Katheter mit dessen proximalem Ende drehfest kuppelbar ist.

4. Katheter nach Anspruch 1 oder 2, dadurch gekennzeichnet, daß ein Antriebsmotor (5) für den Drehantrieb des Katheters (1) und des Stilettes (7) zwei in Flucht miteinander angeordnete Abtriebswellen (10) hat, die hohl sind, wobei das Stilett (7) durch beide Abtriebswellen in den mit der ersten Abtriebswelle drehfest gekuppelten Katheter einführbar und in Gebrauchsstellung mit der zweiten Abtriebswelle lösbar kuppelbar ist.

5. Katheter nach einem der Ansprüche 1 bis 4, dadurch gekennzeichnet, daß das distale Ende (8) des Stilettes (7) einen Anschlag beispielsweise eine Kugel oder dergleichen zum Festlegen gegen einen am distalen Ende, insbesondere im Inneren des Druckkörpers (3) befindlichen Gegenanschlag oder Absatz (14) aufweist.

6. Katheter nach einem der Ansprüche 1 bis 5, dadurch gekennzeichnet, daß das Stilett (7) bei Normaltemperatur gerade ist, zumindest aber im Bereich seines distalen Endes am Ort der Vorbiegung oder Vorkrümmung (9) aus Memory-Metall besteht und bei Körpertemperatur gebogen oder gekrümmt ist.

7. Katheter nach Anspruch 1 bis 6, dadurch gekennzeichnet, daß das Stilett (7) oder dergleichen in einer gegenüber dem distalen Ende etwas zurückgezogenen Position mit dem Drehantrieb (5) kuppelbar ist und sein durch die Vorkrümmung (9) ausgelenktes Ende den dem Druckkörper (3) des Katheters (1) benachbarten biegsamen oder flexiblen Bereich des Katheters (1) gegenüber dessen Verlauf dieses Katheters und gegenüber der mittleren Position des Druckkörpers (3) auslenkt und bei Rotation des Stilettes (7) in eine etwa kreisförmige Bewegung versetzt, und daß an diesem Bereich vorzugsweise ein zweiter Druckkörper (16) an der Außenseite des Katheters (1) fest oder lösbar angeordnet ist.

8. Katheter nach einem der Ansprüche 1 bis 7, dadurch gekennzeichnet, daß der Katheter (1) eine einlagige und vorzugsweise ein- oder mehrgängige Wendel aus flexiblem Metalldraht, von Kunststoffäden und/oder Bändern ist.

9. Katheter nach einem der Ansprüche 1 bis 8, dadurch gekennzeichnet, daß die nebeneinanderliegenden schraubenlinienförmigen Windungen (17) zumindest in den geraden Bereichen und an der Innenseite einer Krümmung einander berühren.

10. Katheter nach einem der Ansprüche 1 bis 9, dadurch gekennzeichnet, daß der Katheter aus mehreren parallel gewundenen und/oder sich kreuzenden Drähten, Fäden und/oder Bändern geflochten ist.

11. Katheter nach einem der Ansprüche 1,2 oder 5 bis 10, dadurch gekennzeichnet, daß auch der Druckkörper drehbar gelagert ist.

12. Katheter nach Anspuch 11, dadurch gekennzeichnet, daß der Druckkörper über ein drehbares Element mit dem Drehantrieb kuppelbar ist.

## Claims

1. A dilatation catheter (1) for reducing or eliminating vasoconstrictions or vascular occlusions (2), having fitted to the distal end thereof a pressure member (3) serving to exert a pressure on the constricted vascular wall, **characterized in that** the catheter (1) has an inner guide channel (6) for a stylet (7) and is flexibly bendable or deflectable near the pressure member (3), that the stylet (7) has as a support and control element near its end (8) projecting up to or into the pressure member (3) in the position of use a pre-bend or pre-curvature (9) and that the stylet (7) is adapted to be coupled to and to be rotary driven by a rotary actuator (5) arranged outside the vessel at the proximal end.

2. A catheter as claimed in claim 1, characterized in that the permanent pre-bend or pre-curvature (9) of the stylet (7) is changeable, possibly repeatedly by bending or bending back the stylet more intensively.

3. A catheter as claimed in claim 1 or claim 2, characterized in that the rotary actuator (5) is a motor with a hollow output shaft (10), that the catheter (1) runs through said hollow output shaft and the motor and is adapted to be coupled fast for rotation with the output shaft, and that after having been introduced into the hollow catheter the stylet is adapted to be coupled fast for rotation with the proximal end of said catheter.

4. A catheter as claimed in claim 1 or claim 2, characterized in that a driving motor (5) for the rotary drive of the catheter (1) and stylet (7) has two output shafts (10) which are arranged in alignment with each other and are hollow, the stylet (7) being insertable through both output shafts into the catheter coupled fast for rotation with the first output shaft and being adapted to be separably coupled to the second output shaft in the position of use.

5. A catheter as claimed in any one of claims 1 to 4, characterized in that the distal end (8) of the stylet (7) has a stop, by way of example a ball or the like, for location against a counter-stop or shoulder (14) situated at the distal end, particularly inside the pressure member (3).

6. A catheter as claimed in any one of claims 1 to 5, characterized in that the stylet (7) is straight at normal temperature, but consists of memory metal at least in the area of the distal end thereof at the location of the pre-bend or pre-curvature (9) and is bent or curved at body temperature.

7. A catheter as claimed in claims 1 to 6, characterized in that in a position slightly retracted relative to the distal end the stylet (7) or the like is adapted to be coupled to the rotary actuator (5), and the stylet end deflected through the pre-curvature (9) deflects the pliant or flexible catheter (1) zone adjacent to the pressure member (3) of the catheter (1) relative to the path of said catheter and relative to the mid position of the pressure member (3) and sets the same into an approximately circular movement as the stylet (7) rotates, and that at said zone preferably a second pressure member (16) is permanently or detachably arranged on the outside of the catheter (1).

8. A catheter as claimed in any one of claims 1 to 7, characterized in that the catheter (1) is a single-layer and preferably single or multiple helix of flexible metallic wire, plastic filaments and/or bands.

9. A catheter as claimed in any one of claims 1 to 8, characterized in that the adjacent turns (17) in the shape of a helical line touch each other at least in the straight zones and at the inside of a curvature.

10. A catheter as claimed in any one of claims 1 to 9, characterized in that the catheter is interlaced from a plurality of parallel wound and/or crossing wires, filaments and/or bands.

11. A catheter as claimed in any one of claims 1, 2 or 5 to 10, characterized in that the pressure member is also rotatable.

12. A catheter as claimed in claim 11, characterized in that the pressure member is adapted to be coupled to the rotary actuator by way of a rotatable element.

## Revendications

1. Cathéter de dilatation (1) pour réduire ou supprimer des rétrécissements ou occlusions (2) de vaisseaux sanguins, avec un corps de pression (3), disposé à son extrémité distale, qui sert à exercer une pression sur la cloison rétrécie du vaisseau sanguin, **caractérisé** en ce que le cathéter (1) présente un canal de guidage intérieur (6) pour un stylet (7) et peut être plié ou dévié de façon flexible au voisinage du corps de pression (3), en ce que le stylet (7) présente une cambrure ou courbure préalable (9) comme élément de soutien et de commande au voisinage de son extrémité (8) qui, en position d'utilisation, atteint le corps de pression (3) ou y pénètre, et en ce que le stylet (7) peut être accouplé à un entraînement en rotation (5) disposé en dehors du vaisseau sanguin à l'extrémité proximale, et peut ainsi être entraîné en rotation.

2. Cathéter selon la revendication 1, **caractérisé** en ce que la cambrure ou courbure préalable permanente (9) du stylet (7) peut être modifiée, éventuellement de façon répétée, par une plus grande flexion ou une flexion inverse.

3. Cathéter selon la revendication 1 ou 2, **caractérisé** en ce que l'entraînement en rotation (5) est un moteur avec un arbre de sortie creux (10), en ce que le cathéter (1) traverse cet arbre de sortie creux ainsi que le moteur et peut être accouplé en solidarité de rotation à l'arbre de sortie, et en ce que le stylet peut, à la suite de son introduction dans le cathéter creux, être accouplé en solidarité de rotation à l'extrémité proximale de ce dernier.

4. Cathéter selon la revendication 1 ou 2, **caractérisé** en ce qu'un moteur d'entraînement (5) pour l'entraînement en rotation du cathéter (1) et du stylet (7) possède deux arbres de sortie (10) disposés en alignement mutuel qui sont creux, le stylet (7) pouvant être introduit en traversant les deux arbres de sortie dans le cathéter accouplé en solidarité de rotation au premier arbre de sortie et pouvant, en position d'utilisation, être accouplé de façon amovible au second arbre de sortie.

5. Cathéter selon l'une des revendications 1 à 4, **caractérisé** en ce que l'extrémité distale (8) du stylet (7) présente une butée, par exemple une boule ou similaire, pour sa fixation en position contre une butée complémentaire ou décrochement (14) présent à l'extrémité distale, notamment à l'intérieur du corps de pression (3).

6. Cathéter selon l'une des revendications 1 à 5, **caractérisé** en ce que le stylet (7) est rectiligne à la température normale, mais est réalisé en métal à mémoire au moins dans la région de son extrémité distale, à l'endroit de la cambrure ou courbure préalable (9), et est cambré ou courbé à la température du corps.

7. Cathéter selon l'une des revendications 1 à 6, **caractérisé** en ce que le stylet (7) ou similaire peut être accouplé à l'entraînement en rotation (5) dans une position légèrement en retrait par rapport à l'extrémité distale, et son extrémité déviée par la courbure préalable (9) dévie la région souple ou flexible du cathéter (1), voisine du corps de pression (3) du cathéter (1), par rapport à l'allure de ce cathéter et par rapport à la position médiane du corps de pression (3) et, lors de la rotation du stylet (7), met cette région en un mouvement approximativement circulaire, et en ce que, de préférence, un second corps de pression (16) est disposé de manière fixe ou amovible dans cette région, sur le côté extérieur du cathéter (1).

8. Cathéter selon l'une des revendications 1 à 7, **caractérisé** en ce que le cathéter (1) est un boudin monocouche et de préférence à enroulement simple ou multiple, constitué de fil métallique flexible, de fils de matière synthétique et/ou de bandes.

9. Cathéter selon l'une des revendications 1 à 8, **caractérisé** en ce que les enroulements hélicoïdaux voisins (17) se touchent mutuellement dans les régions droites et sur le côté intérieur d'une courbure.

10. Cathéter selon l'une des revendications 1 à 9, **caractérisé** en ce que le cathéter est tressé à partir de plusieurs fils métalliques, fils synthétiques et/ou bandes enroulés en parallèle et/ou croisés.

11. Cathéter selon l'une des revendications 1, 2 ou 5 à 10, **caractérisé** en ce que le corps de pression est lui aussi monté à rotation.

12. Cathéter selon la revendication 11, **caractérisé** en ce que le corps de pression peut être accouplé à l'entraînement en rotation par l'intermédiaire d'un élément rotatif.
